# EUROPEAN PATENT SPECIFICATION

(11) **EP 3 581 212 B1**
(45) Date of publication and mention of the grant of the patent: **06.08.2025**
(21) Application number: 18177029.8
(22) Date of filing: 11.06.2018
(51) Int. Cl.: A61L 27/26, A61L 27/56

(54) **FIBROUS MATERIAL AND ITS PREPARATION**
FASERMATERIAL UND DESSEN HERSTELLUNG
MATÉRIAU FIBREUX ET SA PRÉPARATION

(43) Date of publication of application: 18.12.2019
(73) Proprietor: Fraunhofer-Gesellschaft zur Förderung der angewandten Forschung e.V., 80686 München (DE)
(72) Inventor: Schmelzer, Christian, 06114 Halle (Saale) (DE); Hedtke, Tobias, 06114 Halle (Saale) (DE)
(74) Representative: Friese Goeden Patentanwälte PartGmbB

(56) References cited:
- WO-A1-2008/037028
- US-A1- 2006 263 417
- BUTTAFOCO L ET AL: "Electrospinning of collagen and elastin for tissue engineering applications", BIOMATERIALS, ELSEVIER SCIENCE PUBLISHERS BV., BARKING, GB, vol. 27, no. 5, 1 February 2006 (2006-02-01), pages 724 - 734, XP027950845, ISSN: 0142-9612, [retrieved on 20060201]
- BOLAND E D ET AL: "ELECTROSPINNING COLLAGEN AND ELASTIN: PRELIMINARY VASCULAR TISSUE ENGINEERING", FRONTIERS IN BIOSCIENCE, FRONTIERS IN BIOSCIENCE, ALBERTSON, NY, US, vol. 9, 1 May 2004 (2004-05-01), pages 1422 - 1432, XP008061377, ISSN: 1093-9946

## Description

The present invention relates to a fibrous material, uses of the fibrous material and a method for preparing the fibrous material.

Elastin is a biopolymer present in the extracellular matrix (ECM) of vertebrates and provides elasticity and resilience to tissues and organs. Elastin has outstanding characteristics such as a very low elastic modulus, a very high biological half-life and a high resistance to proteolytic enzymes. Thus, it is an ideal model for elastomeric protein-based biomaterials. There is a great demand for tunable biomaterials in medicine that are elastic, biocompatible, biodegradable and absorbable, e.g. for wound-treatment, tissue replacement or implant coating. A promising method to fabricate such biomaterials is electrospinning, which is a fiber-forming process utilizing an electric field. A high voltage is applied between a grounded collector and a capillary containing a polymer solution (which can be a protein or peptide solution) or a wire that is continuously wetted with this solution. Spinneret (the capillary or wire) drops are formed from which electrified thin fluid jets are then ejected. The jet elongates, thins and eventually dries and solidifies while it moves to the collector. The fibers are deposited on the collector as a nonwoven mat, in which the fibers show a random orientation. The resultant fibers usually have diameters in the range of nm to µm and can mimic the fibrous structure of the ECM to some extent.

Elastin isolated from vertebrate tissues (e. g. aorta, skin) is insoluble in all organic and inorganic solvents and therefore *per se* incompatible with the above-described method of electrospinning. Further processing is necessary in order to transform the macromolecular elastin in a soluble derivative with similar properties. Also the use of processed elastin for the development of fibrous, film- and gel-like biomaterials is already described (see below). However, to date none of the methods reported in the literature describes the use of a multi-component elastin-based material blend for the production of tunable fibrous biomaterials. This is reasonable as it is challenging to produce highly porous fibrous materials with a sufficient elastin percentage without any auxiliary polymers of non-biological origin.

Up to the date, the development and production of elastin-based biomaterials by electrospinning was limited to the lab scale and the elastin components were in a low percentage or blended with synthetic materials (e. g. polylactic-co-glycolic acid) to gain fibers with diameters below 1 µm (Co-Electrospun Blends of PLGA, Gelatin, and Elastin as Potential Nonthrombogenic Scaffolds for Vascular Tissue Engineering. Jingjia Han, Philip Lazarovici, Colin Pomerantz, Xuesi Chen, Yen Wei, Peter I. Lelkes. 2, 2011, Biomacromolecules, Vol. 12, p. 399 - 408; Electrospun polydioxanone-elastin blends: potential for bioresorbable vascular grafts. S. A. Sell, M. J. McClure, C. P. Barnes, D. C. Knapp, B. H. Walpoth, D. G. Simpson and G. L. Bowlin, 2006, Biomedical Materials, Vol. 1.). An overview of different fibrous material blends containing elastin is given in a review by Yeo et al. Fabricated Elastin, Giselle C. Yeo, Behnaz Aghaei-Ghareh-Bolagh, Edwin P. Brackenreg , Matti A. Hiob , Pearl Lee, Anthony S. Weiss 16, 2015, Advanced Healthcare, Vol. 4, pp. 2530 - 2556. The use of pure elastin hydrolysates or recombinant tropoelastin was demonstrated in lab scale without or only limited tunable properties (Structural and Cellular Characterization of Electrospun Recombinant Human Tropoelastin Biomaterials. Kathryn A. McKenna, Kenton W. Gregory, Rebecca C. Sarao, Cheryl L. Maslen, Robert W. Glanville, Monica T. Hinds. 2, 2012, Journal of Biomaterials Applications, Vol. 27, pp. 219-230; Processing and characterization of α-elastin electrospun membranes. J. Araujo, J. Padrao, J. P. Silva, F. Dourado, D. M. Correia, G. Botelho, J. L. Gomez Ribelles, S. Lanceros-Méndez, V. Sencadas. 4, 2014, Applied Physics A, Vol. 115, pp. 1291-1298). In addition, wet spinning was applied to biomolecules resulting in fibers with a diameter of several micrometers depending on the material used (Wet-Spinning of Recombinant Silk~Elastin-Like Protein Polymer Fibers with High Tensile Strength and High Deformability. Weiguo Qiu, Weibing Teng, Joseph Cappello, Xiaoyi Wu. 3, 2009, Biomacromolecules, Bd. 10, pp. 602-608; Properties of fibers produced from soy protein isolate by extrusion and wet-spinning. H. C. Huang, E. G. Hammond, C. A Reitmeier, D. J. Myers. 12, 1995, Journal of the American Oil Chemists' Society, Vol. 72, pp. 1453-1460). E.D. Boland, J.A. Matthews, K.J. Pawlowski, D.G. Simpson, G.E. Wnek, G.L. Bowlin: "Electrospinning collagen and elastin: preliminary vascular tissue engineering", Front. Biosci. 9 (2004) 1422 discloses the electrospinning of micro- and nano-fibrous scaffoldings from the natural polymers collagen and elastin and its application to development of biomimicking vascular tissue engineered constructs.

So far, no scalable process for the production of tunable elastin-based materials with a high elastin content without using polymers of non-biological origin is described.

The technical problem underlying the present invention is to provide a fibrous material, the use of this material and a method for producing fibrous material avoiding the above drawbacks. In particular, it is an object to produce fibrous materials, which are tunable and contain a sufficient elastin amount without any auxiliary polymers of non-biological origin.

This object has been solved by the fibrous material of claim 1, the multi-layer material of claim 7, the biomaterial of claim 8, the implantable medical device of claim 9, the medical device for wound treatment of claim 10 and the method for preparing the fibrous material by electrospinning according to claim 11. Embodiments of the present invention are defined in the dependent claims.

The term "elastin" refers to elastin of any kind, in particular to any natural or recombinant or chemically synthesized protein, isolated natural biopolymer, genetically engineered protein construct and all processed forms (e.g. partial hydrolysates) which have corresponding properties to native elastin in terms of the amino acid sequence, repetitive amino acid entities as well as mechanical, chemical, biological and biochemical properties.

According to the present invention, a fibrous material containing compounds (a) and (b) is concerned. This means that each fiber of the fibrous material contains elastin as compound (a) together with the material of biological origin as compound (b) in the indicated amounts. Therefore, the fibrous material comprises or consist of a mixture of the compounds (a) and (b). As to elastin, it is present in an amount of 75 wt% or more, referring to the fibrous material, but since also a material of biological origin is present in an amount greater 0 wt%, the amount of elastin is less than 100 wt% in the fibrous material.

The fibrous material according to the present invention contains nano- and/or microfibers and has elastic properties. It is also stable in aqueous conditions and shows elevated resistance against proteolytic enzymes, for example trypsin. Furthermore, the fibrous material has a porous structure and is a non-woven material.

In a preferred embodiment, the elastin is selected from the group of hydrolyzed elastin, recombinant elastin, recombinant and/or synthetic elastin-like polypeptide comprising at least one lysine residue and repetitive motifs of the sequence Val-Pro-Gly-Xaa-Gly, wherein Xaa is any canonical amino acid except Pro. In a further embodiment, the hydrolyzed elastin is selected from the group of α-elastin and K-elastin. A mixture of any of the above substances can be used in the fibrous material according to the present invention.

In one embodiment of the present invention, the diameter of the fibers of the fibrous material can range from 80 nm to 1400 nm. The diameters of the fibers can be measured by scanning electron microscopy (SEM).

Furthermore, in one embodiment the fibrous material is water-stable, which means that the fibers do not disintegrate in water; the fiber structure is maintained. The term "stable" refers to the fact that for at least 24 hours at room temperature not more than 10 % of the fibrous material in water is disintegrated.

The water stability of the fibrous material can be obtained by covalent cross-linking of the fibers. Covalent cross-linking can be obtained by employing at least one cross-linking agent or at least one cross-link inducing agent selected from the group of aldehydes, dialdehydes, carbodiimides, N-hydroxysuccinimide esters, N-hydroxysulfosuccinimide esters, epoxids, anhydrids, acyl azides, sulfonyl chlorides, isocyanates, isothiocyanats, genipin, allyl-containing agents, radical initiators, and ortho-quinones or polyphenols in combination with divalent metal ions. An example suitable as an aldehyde for cross-linking is formaldehyde. An example of a dialdehyde is glutardialdehyde. Furthermore, covalent cross-linking can be obtained because of enzymatic activity.

As mentioned above, the amount of the material of biological origin is greater 0 wt% to 25 wt%, referring to the fibrous material. In one embodiment, the amount of the material of biological origin is 10 wt% to25 wt%, referring to the fibrous material. The amount of elastin is 75 wt% or more to less than 100 wt% referring to the fibrous material.

For example, the fibrous material according to the present invention comprises or consists of κ-elastin in an amount of at least 75 wt% or more, referring to the fibrous material, and gelatin and/or collagen.

As compound (b) a material of biological origin is present in the fibrous material according to the present invention. As material of biological origin gelatin is used.

The fibrous material according to the present invention can be obtained by electrospinning. Electrospinning is a process for preparing fibers known in the art so that the skilled person knows the parameters and the materials for conducting electrospinning. Electrospinning is different from wet spinning, in that wet spinning provides significantly thicker fibers than electrospinning. Fibers obtained by wet spinning have diameters of several micrometers in contrast to fibers obtained by electrospinning where the majority of the fibers have diameters of less than 1 µm.

Further provided is a multi-layer material containing at least two layers wherein each of the two layers is made from the fibrous material according to the present invention described above in detail. The composition of the at least two layers can be identical or different from each other.

The at least two layers in the multi-layer material can be connected to each other in particular in such a way that they cannot be peeled off from each other manually. This connection of the at least two layers can be obtained by chemical or photochemical cross-linking. It is clarified that this chemical or photochemical cross-linking is a further cross-linking to be carried out in addition to the above-mentioned covalent cross-linking to obtain water stability. The covalent cross-linking of the at least two layers to each other can be carried out for example using formaldehyde. Alternatively, to connect the at least two layers to each other laser welding can be used.

In addition to the two layers each made from fibrous material according to the present invention a further layer of a single-component electrospun nonwoven material can be present in the multi-layer material of the present invention. That is, in this case the multi-layer material can contain three layers, namely two layers of the fibrous material and one further layer. The single component material can be selected from the group of gelatin, collagen, silk fibroin, alginin or chitosan.

The fibrous material according to the present invention and the multi-layer material according to the present invention provide several advantages. Multi-component protein blends with a high elastin percentage are provided that can be used for the production of nonwoven materials by electrospinning. The materials according to the present invention are compatible with small industrial scale production of nonwoven materials by electrospinning. The materials according to the present invention contain a high percentage of elastin and preserve the fibrous structure under aqueous condition. The raw materials can be obtained from byproducts of the food industry and are therefore inexpensive and available in large quantities. The materials according to the present invention have elastic properties and are tunable in the sense that the properties, in particular the elastic properties, can be adjusted as required for the end-use. These advantages are in particular obtainable by the above-defined embodiments of the fibrous material and the multi-layer fibrous material according to the present invention.

The above-defined properties of the fibrous material and the multi-layer material according to the present invention make these materials suitable among others in the following fields: biomedicine, surgery, wound treatment, medical devices, implant coating, cell culturing, and tissue engineering. These are known technical applications for various materials, in particular biomaterials (i.e. materials which can be brought into contact with biological systems such as the human body). Therefore, the skilled person knows methods and materials how to use the fibrous material according to the present invention and the multi-layer material according to the present invention in the above fields.

In one embodiment, the fibrous material according to the present invention and the multi-layer material according to the present invention can contain cells, so that a cell-seeded material is provided. The cells can be provided on the surface of the material, in particular by applying known techniques. The cells can be in particular human cells so that the material seeded with the cells can be used for human compatible artificial tissues and organs.

In a further embodiment, the fibrous material according to the present invention and the multi-layer material according to the present invention can contain drugs. The drug can be provided on the surface of the material by applying known techniques. The term "drug" refers to any compound causing a temporary physiological change in the body when administered. Such drugs can be used for treating diseases. The drug-loaded material can be produced by coaxial electrospinning in which the drug is incorporated into the fibers. The drug-loaded fibrous material or the drug-loaded multi-layer material can be degraded in the body so that the drug is liberated into the body.

In a further embodiment, the present invention provides a biomaterial made from or containing the fibrous material according to the present invention or the multi-layer material according to the present invention. A biomaterial is a material used in medicine for diagnostic or therapeutic purpose and which comes into contact with the biological tissue of the body.

Further, an implantable medical device made from or containing the fibrous material according to the present invention or the multi-layer material according to the present invention is provided. Such an implantable medical device can comprise a surface-activated material of non-biological origin and at least one covalently or non-covalently immobilized material selected from the fibrous material according to the present invention and/or the multi-layer material according to the present invention. The fibrous material or multi-layer material used in the implantable material according to the present invention can be chemical modified to improve the adhesive strength. The aforementioned material of non-biological origin means any material which stems not from biological material. An example of the material of non-biological origin is polyether ether ketone (PEEK). The above-mentioned surface activation can be achieved by plasma treatment or any other known method in this field. Furthermore, the implantable medical device can contain drugs as mentioned above, for example analgesics and/or antibiotics.

Furthermore, a medical device for wound treatment is provided which can be made from or can contain the fibrous material according to the present invention or the multi-layer material according to the present invention. The material can be immobilized onto an adhesive-containing carrier made of a material of non-biological origin. The material of non-biological origin can be defined as above. Examples of such a material of non-biological origin are polymers used in the field of wound treatment.

There is also provided a method for preparing the fibrous material according to the present invention as described in detail above by electrospinning.

Electrospinning is a method known for producing biomaterials so that the skilled person does know materials and parameters for carrying out electrospinning. Briefly, electrospinning is a fiber-forming process utilizing an electric field. A high voltage is applied between a grounded collector and a capillary containing the solution of components (a) and (b) mentioned above or a wire that is continuously wetted with this solution. At the spinneret (the capillary or wire), drops are formed from which electrified thin fluid jets are then ejected and solidified while they move to the collector. The fibers are deposited on the collector as a nonwoven mat, in which the fibers show a random orientation. The resultant fibers usually have diameters in the range of nm to µm and can mimic the fibrous structure of the extracellular matrix (ECM) to some extent. By applying electrospinning, a scalable process for the production of elastin-based materials is provided, with which the properties of the fibrous material can be tuned and a high elastin content without using polymers of non-biological origin can be obtained. Furthermore, electrospinning is applicable to a small production line as well as an industrial production line.

Mature elastin can be isolated from vertebrate aorta tissue by hot alkaline treatment as known in this field. α-elastin and K-elastin can subsequently be produced by treatment with 0.25 M oxalic acid or in an alcoholic solution of 1 M potassium hydroxide as known in the art. α- and K-elastin can be subsequently blended with the material of biological origin as defined above in the indicated amounts. For the application of the present invention, two- or three component blends can be used. The blend can contain 75 wt% K-elastin and/or α-elastin and 25 wt% gelatin and/or collagen. The protein blends are solubilized in formic acid or acetic acid, in particular in a mixture of equal volumes of both in a total concentration of 15 - 25 % (w/v), for example 20 % (w/v). After electrospinning, the mats are stabilized by chemical cross-linking as mentioned above. Volatile cross-linking agents like formaldehyde and glutaraldehyde can be used to be applied to the nonwoven in the gaseous phase. Other cross-linkers may be applied in the liquid phase using organic solvents like diethyl ether or isopropanol. The resulting fibrous material contains nano- and microfibers and has elastic properties. It is also stable in aqueous conditions and shows elevated resistance against proteolytic enzymes such as trypsin.

An electrospinning process can be carried out according to the following illustrative protocol: Fresh porcine aorta was obtained from a local slaughterhouse and stored at -80 °C until use. For the isolation of elastin the tissue was thawed in 0.9 % NaCl and cleared from non-aortic remains. The tissues was chopped and subsequently frozen in liquid nitrogen to facilitate the following pulverization step. 250 mL to 350 mL (i.e. 120 g to 180 g) of pulverized aortic tissue were extracted two times with 1M NaCl containing 0.02 % NaN₃ for 1 h at room temperature under permanent stirring. The washed tissue was subsequently defatted by incubation in 700 mL EtOH and 700 mL acetone for 1 h each. The defatted tissue was dried overnight and hydrolyzed the next day by 0.1 M NaOH for 45 min. The remaining elastin pellet was rinsed with EtOH and dried overnight. κ-elastin was produced by partial hydrolysis of the isolated elastin in 1M NaOH dissolved in 80 % EtOH. For each 10 g of elastin 250 mL of 1M NaOH was used. Afterwards the pH was lowered to 11.5 and the κ-elastin solution was dialyzed against doubly distilled water for 48 h prior to lyophilization. The electrospinnable blend solution was prepared by dissolving 6g κ-elastin and 2 g gelatin in 40 mL of a 1:1 mixture of formic and acetic acid. The solution was stirred for 3 h. The solution was subsequently transferred to the electrospinning device (Elmarco Nanospider NS 1S500U). The process parameters for electrospinning were:
Collector electrode current: 20 kV
Donor electrode current: 60 kV
Setup: Wire-setup
Temperature: 25.3 °C
Relative humidity: 22.6 %
Duration of spinning: 4x 15 min @ 15 mm/min flow rate of the collector fleece (4 layers were spun on top of each other).

The nonwovens were cut in pieces of 10x10 cm² and cross-linked by gassing with formaldehyde. For this 25 mL per 100 mg fleece of a 37 % formaldehyde solution were filled into a glass container and placed in the bottom of a desiccator. The nonwovens were placed above the container and the samples were incubated for 90 min at room temperature in a tightly closed desiccator.

The present invention is further illustrated by the following examples and by reference to the following figures, wherein neither the examples nor the figures shall be construed to limit the invention thereto.

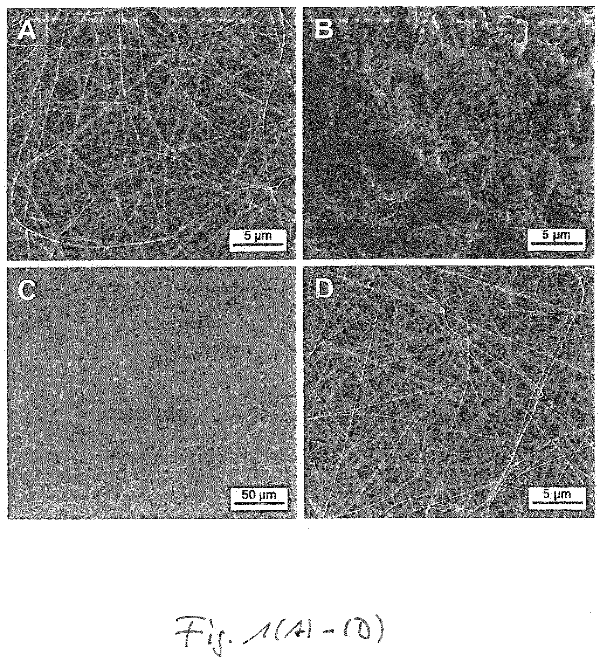

FIG. 1 (A) to (D) shows scanning electron micrographs of a formaldehyde cross-linked fibrous material containing 75 % K-elastin before (A) and after (B) incubation in distilled water for 24 hours; a non-cross-linked fibrous material containing 50 % α-elastin is shown in (C) and (D) in two different magnifications.

### Example 1: Preparation of formaldehyde cross-linked fibrous material containing 75 % κ-elastin

Fresh porcine aorta was obtained from a local slaughterhouse and stored at -80 °C until use. For the isolation of elastin, the tissue was thawed in 0.9 % NaCl and cleared from non-aortic remains. The tissues were chopped and subsequently frozen in liquid nitrogen to facilitate the following pulverization step. 250 mL to 350 mL (i.e. 120 g to 180 g) of pulverized aortic tissue were extracted two times with 1 M NaCl containing 0.02 % NaN₃ for 1 h at room temperature under permanent stirring. The washed tissue was subsequently defatted by incubation in 700 mL EtOH and 700 mL acetone for 1 h each. The defatted tissue was dried overnight and hydrolyzed the next day by 0.1 M NaOH for 45 min. The remaining elastin pellet was rinsed with EtOH and dried overnight.

κ-elastin was produced by partial hydrolysis of the isolated elastin in 1 M NaOH dissolved in 80 % EtOH. For each 10 g of elastin 250mL of 1 M NaOH was used. Afterwards the pH was lowered to 11.5 and the κ-elastin solution was dialyzed against doubly distilled water for 48 h prior to lyophilization. The electrospinnable blend solution was prepared by dissolving 6 g κ-elastin and 2 g gelatin in 40 mL of a 1:1 mixture of formic and acetic acid. The solution was stirred for 3 h. The solution was subsequently transferred to the electrospinning device (Elmarco Nanospider NS 1S500U). The process parameters for electrospinning were:
Collector electrode current: 20 kV
Donor electrode current: 60 kV
Setup: Wire-setup
Temperature: 25.3 °C
Relative humidity: 22.6 %
Duration of spinning: 4x 15 min @ 15 mm/min flow rate of the collector fleece (4 layers were spun on top of each other).

The nonwovens were cut in pieces of 10x10 cm² and cross-linked by gassing with formaldehyde. For this 25 mL per 100 mg fleece of a 37 % formaldehyde solution were filled into a glass container and placed in the bottom of a desiccator. The nonwovens were placed above the container and the samples were incubated for 90 min at room temperature in a tightly closed desiccator.

FIG. 1 (A) and 1 (B) show scanning electron micrographs of a formaldehyde cross-linked fibrous material containing 75 % κ-elastin obtained as described above before (A) and after (B) incubation in distilled water for 24 hours 1.

### Example 2: Preparation of a non-cross-linked fibrous material containing 50 % α-elastin (not according to the claims)

Freshg porcine aorta was obtained from a local slaughterhouse and stored at -80 °C until use. For the isolation of elastin, the tissue was thawed in 0.9 % NaCl and cleared from non-aortic remains. The tissues were chopped and subsequently frozen in liquid nitrogen to facilitate the following pulverization step. 250 mL to 350 mL (i.e. 120 g to 180 g) of pulverized aortic tissue were extracted two times with 1 M NaCl containing 0.02 % NaN₃ for 1 h at room temperature under permanent stirring. The washed tissue was subsequently defatted by incubation in 700 mL EtOH and 700 mL acetone for 1 h each. The defatted tissue was dried overnight and hydrolyzed the next day by 0.1 M NaOH for 45 min. The remaining elastin pellet was rinsed with EtOH and dried overnight.

α-elastin was produced by partial hydrolysis of the isolated elastin in 0.25 M oxalic acid. The α-elastin solution was dialyzed against doubly distilled water for 48 h prior to lyophilization.

The electrospinnable blend solution was prepared by dissolving α-elastin and gelatin to equal parts in formic acid at a total protein concentration of 22.5 % (w/v). The solution was subsequently stirred overnight. Electrospinning was performed on an experimental device equipped with a "Heinzinger PNC 30000-5 pos" high voltage power supply and a "NEMESYS NEM-B101-02 C" syringe pump. The needle diameter was 0.4 mm, the voltage was adjusted to 30kV and the flow rate was set to 5 µL/min.

Fig 1(C) and Fig. 1(D) shown the respectively obtained non-cross-linked fibrous material in two different magnifications.

## Claims

1. A fibrous material comprising:
(a) elastin in an amount of 75 wt% or more, referring to the fibrous material, and
(b) a material of biological origin in an amount of greater 0 wt% to 25 wt%, referring to the fibrous material
wherein the fibrous material is obtainable by electrospinning an electrospinnable blend solution containing the elastin and the material of biological origin in formic acid and/or acetic acid without an auxiliary polymer of non-biological origin, and
wherein the material of biological origin is gelatin.

2. The fibrous material of claim 1, wherein the elastin is selected from the group of hydrolyzed elastin, recombinant elastin, recombinant elastin-like polypeptide comprising at least one lysine residue and repetitive motifs of the sequence Val-Pro-Gly-Xaa-Gly, wherein Xaa is any canonical amino acid except Pro.

3. The fibrous material of claim 2, wherein the hydrolyzed elastin is selected from the group of α-elastin and κ-elastin.

4. The fibrous material of any of the preceding claims, wherein a diameter of fibers of the fibrous material ranges from 80 nm to 1400 nm.

5. The fibrous material of any of the preceding claims wherein water stability of the fibrous material is obtained by covalent cross-linking of the fibrous material.

6. The fibrous material of claim 5, wherein covalent cross-linking is obtained by employing at least one cross-linking agent or at least one cross-link inducing agent selected from the group of aldehydes, dialdehydes, carbodiimides, N-hydroxysuccinimide esters, N-hydroxysulfosuccinimide esters, epoxids, anhydrids, acyl azides, sulfonyl chlorides, isocyanates, isothiocyanats, genipin, allyl-containing agents, radical initiators, and ortho-quinones or polyphenols in combination with divalent metal ions and/or wherein the covalent cross-linking is obtained by enzymatic activity.

7. A multi-layer material containing at least two layers each made from the fibrous material of any of claims 1 to 6.

8. The fibrous material of any of claims 1 to 6 or the multi-layer material of claim 7 containing cells and/or drugs.

9. An implantable medical device made from or containing the fibrous material of any of claims 1 to 6, or the multi-layer material of claim 7 or 8.

10. A medical device for wound treatment made from or containing the fibrous material of any of claims 1 to 6, or the multi-layer material of claim 7 or 8.

11. Method for preparing the fibrous material of any of claims 1 to 6 comprising the step of electrospinning an electrospinnable blend solution containing the elastin and the material of biological origin in formic acid and/or acetic acid without an auxiliary polymer of non-biological origin, said blend solution containing
(a) elastin in an amount of 75 wt% or more, referring to the fibrous material, and
(b) the material of biological origin in an amount of greater 0 wt% to 25 wt%, referring to the fibrous material, and
wherein the material of biological origin is gelatin.

## Patentansprüche

1. Fasermaterial, umfassend:
(a) Elastin in einer Menge von 75 Gew.-% oder mehr, bezogen auf das Fasermaterial, und
(b) ein Material biologischen Ursprungs in einer Menge von mehr als 0 Gew.-% bis 25 Gew.-%, bezogen auf das Fasermaterial,
wobei das Fasermaterial durch Elektrospinnen einer elektrospinnbaren Mischlösung erhältlich ist, die das Elastin und das Material biologischen Ursprungs in Ameisensäure und/oder Essigsäure ohne ein Hilfspolymer nicht-biologischen Ursprungs enthält, und
wobei das Material biologischen Ursprungs Gelatine ist.

2. Fasermaterial nach Anspruch 1, wobei das Elastin aus der Gruppe von hydrolysiertem Elastin, rekombinantem Elastin, rekombinantem elastinähnlichem Polypeptid, das mindestens einen Lysinrest und repetitive Motive der Sequenz Val-Pro-Gly-Xaa-Gly umfasst, wobei Xaa eine beliebige kanonische Aminosäure mit Ausnahme von Pro ist, ausgewählt ist.

3. Fasermaterial nach Anspruch 2, wobei das hydrolysierte Elastin aus der Gruppe von α-Elastin und κ-Elastin ausgewählt ist.

4. Fasermaterial nach einem der vorhergehenden Ansprüche, wobei der Durchmesser der Fasern des Fasermaterials im Bereich von 80 nm bis 1400 nm liegt.

5. Fasermaterial nach einem der vorhergehenden Ansprüche, wobei die Wasserstabilität des Fasermaterials durch kovalente Vernetzung des Fasermaterials erhalten wird.

6. Fasermaterial nach Anspruch 5, wobei die kovalente Vernetzung durch Verwendung mindestens eines Vernetzungsmittels oder mindestens eines vernetzungsinduzierenden Mittels erhalten wird, das aus der Gruppe der Aldehyde, Dialdehyde, Carbodiimide, N-Hydroxysuccinimidester, N-Hydroxysulfosuccinimidester, Epoxide, Anhydride, Acylazide, Sulfonylchloride, Isocyanate, Isothiocyanate, Genipin, Allyl-enthaltenden Mittel, Radikalinitiatoren und ortho-Chinone oder Polyphenole in Kombination mit zweiwertigen Metallionen ausgewählt ist, und/oder wobei die kovalente Vernetzung durch enzymatische Aktivität erhalten wird.

7. Mehrschichtmaterial, das mindestens zwei Schichten enthält, die jeweils aus dem Fasermaterial nach einem der Ansprüche 1 bis 6 hergestellt sind.

8. Fasermaterial nach einem der Ansprüche 1 bis 6 oder Mehrschichtmaterial nach Anspruch 7, das Zellen und/oder Arzneimittel enthält.

9. Implantierbare medizinische Einrichtung, das aus dem Fasermaterial nach einem der Ansprüche 1 bis 6 oder dem mehrschichtigen Material nach Anspruch 7 oder 8 hergestellt ist oder dieses enthält.

10. Medizinische Einrichtung zur Wundbehandlung, das aus dem Fasermaterial nach einem der Ansprüche 1 bis 6 oder dem mehrschichtigen Material nach Anspruch 7 oder 8 hergestellt ist oder dieses enthält.

11. Verfahren zum Herstellen des Fasermaterials nach einem der Ansprüche 1 bis 6, umfassend den Schritt des Elektrospinnens einer elektrospinnbaren Mischlösung, die das Elastin und das Material biologischen Ursprungs in Ameisensäure und/oder Essigsäure ohne ein Hilfspolymer nicht-biologischen Ursprungs enthält, wobei die Mischungslösung enthält:
(a) Elastin in einer Menge von 75 Gew.-% oder mehr, bezogen auf das Fasermaterial, und
(b) das Material biologischen Ursprungs in einer Menge von mehr als 0 Gew.-% bis 25 Gew.-%, bezogen auf das Fasermaterial, und
wobei das Material biologischen Ursprungs Gelatine ist.

## Revendications

1. Matériau fibreux comprenant :
(a) de l'élastine dans une quantité de 75 % en poids ou plus, par référence au matériau fibreux, et
(b) un matériau d'origine biologique dans une quantité de plus de 0 % en poids à 25 % en poids, par référence au matériau fibreux,
dans lequel le matériau fibreux peut être obtenu par électrofilage d'une solution de mélange électrofilable contenant l'élastine et le matériau d'origine biologique dans de l'acide formique et/ou de l'acide acétique sans polymère auxiliaire d'origine non biologique, et
dans lequel le matériau d'origine biologique est de la gélatine.

2. Matériau fibreux selon la revendication 1, dans lequel l'élastine est sélectionnée parmi le groupe constitué de : élastine hydrolysée, élastine recombinante, polypeptide de type élastine recombinante comprenant au moins un résidu de lysine et des motifs répétitifs de la séquence Val-Pro-Gly-Xaa-Gly, Xaa étant un acide aminé canonique quelconque à l'exception de Pro.

3. Matériau fibreux selon la revendication 2, dans lequel l'élastine hydrolysée est sélectionnée parmi le groupe constitué de : élastine α et élastine κ.

4. Matériau fibreux selon l'une quelconque des revendications précédentes, dans lequel un diamètre de fibres du matériau fibreux va de 80 nm à 1 400 nm.

5. Matériau fibreux selon l'une quelconque des revendications précédentes, dans lequel une stabilité d'eau du matériau fibreux est obtenue par réticulation covalente du matériau fibreux.

6. Matériau fibreux selon la revendication 5, dans lequel une réticulation covalente est obtenue en utilisant au moins un agent de réticulation ou au moins un agent d'induction de réticulation sélectionné parmi le groupe constitué de : aldéhydes, dialdéhydes, carbodiimides, esters de N-hydroxysuccinimide, esters de N-hydroxysulfosuccinimide, époxydes, anhydrides, azotures d'acyle, chlorures de sulfonyle, isocyanates, isothiocyanates, génipine, agents contenant de l'allyle, initiateurs radicaux, et ortho-quinones ou polyphénols en combinaison avec des ions de métal divalents et/ou dans lequel la réticulation covalente est obtenue par activité enzymatique.

7. Matériau multicouche contenant au moins deux couches faites chacune à partir du matériau fibreux selon l'une quelconque des revendications 1 à 6.

8. Matériau fibreux selon l'une quelconque des revendications 1 à 6 ou matériau multicouche selon la revendication 7 contenant des cellules et/ou des médicaments.

9. Dispositif médical implantable fait à partir du ou contenant le matériau fibreux selon l'une quelconque des revendications 1 à 6, ou le matériau multicouche selon la revendication 7 ou 8.

10. Dispositif médical destiné au traitement de plaies fait à partir du ou contenant le matériau fibreux selon l'une quelconque des revendications 1 à 6, ou le matériau multicouche selon la revendication 7 ou 8.

11. Procédé de préparation du matériau fibreux selon l'une quelconque des revendications 1 à 6 comprenant l'étape consistant à électrofiler une solution de mélange électrofilable contenant l'élastine et le matériau d'origine biologique dans un acide formique et/ou un acide acétique sans polymère auxiliaire d'origine non biologique, ladite solution de mélange contenant
(a) de l'élastine dans une quantité de 75 % en poids ou plus, par référence au matériau fibreux, et
(b) le matériau d'origine biologique dans une quantité de plus de 0 % en poids à 25 % en poids, par référence au matériau fibreux, et
dans lequel le matériau d'origine biologique est de la gélatine.
